# EUROPÄISCHE PATENTANMELDUNG

(11) **EP 4 497 810 A1**
(43) Veröffentlichungstag der Anmeldung: **29.01.2025**
(21) Anmeldenummer: 23187568.3
(22) Anmeldetag: 25.07.2023
(51) Int. Cl.: C12M 1/00, C12M 1/21, C12M 1/34, C12M 1/36

(54) **VERFAHREN UND VORRICHTUNG ZUR AUTOMATISIERUNG DER SCHAUMKONTROLLE IN EINEM BIOREAKTOR**

(71) Anmelder: Sartorius Stedim Biotech GmbH, 37079 Göttingen (DE)
(72) Erfinder: KUES, Dominic, 37079 Göttingen (DE); LEUPOLD, Marco, 34302 Guxhagen (DE)
(74) Vertreter: Prinz & Partner mbB

(57) **Zusammenfassung**

Ein Verfahren zur Automatisierung der Schaumregelung während eines Bioprozesses zur Kultivierung von Zellen in einem Bioreaktor umfasst folgende Schritte: Bestimmung von Werten einer oder mehrerer Prozessvariablen des Bioprozesses, die für die Schaumbildung im Bioreaktor relevant ist/sind; Übermitteln der bestimmten Werte als Eingangssignale an einen Controller; Erzeugen von Ausgangssignalen durch den Controller für wenigstens eine Schaumregulierungsvorrichtung in Abhängigkeit der Eingangssignale; und bedarfsgerechte Steuerung der wenigstens einen Schaumregulierungsvorrichtung mittels der vom Controller ausgegebenen Ausgangssignale.

## Beschreibung

Die Erfindung betrifft ein Verfahren zur Automatisierung der Schaumregelung in einem Bioreaktor während eines Bioprozesses zur Kultivierung von Zellen in einem Bioreaktor. Die Erfindung betrifft ferner eine Vorrichtung zur Durchführung dieses Verfahrens.

Ein bekanntes Problem bei Bioprozessen ist die störende Schaumbildung, die verschiedene Ursachen haben kann. Schäume sind u. a. deshalb unerwünscht, da sie die Funktion von Sensoren stören und zu einer Verklebung und Verschmutzung der Reaktorwände führen können, was einen erhöhten Reinigungsaufwand bedingt. Außerdem kann Schaum in Fluidleitungen eindringen, die in den Kopfraum des Bioreaktors münden. Die Verwendung größerer Behälter beseitigt die Probleme nur teilweise und ist zudem unwirtschaftlich. Abgesehen davon ist Schaum in Downstream-Prozessen störend.

Zur Detektion von Schaum in einem Bioreaktor sind verschiedene Techniken bekannt, wie etwa kapazitive Messungen in einem Einweg-Bioreaktor mit einer Patch-Sensoreinheit (siehe z. B. EP 3 872 162 A1) oder Leitfähigkeitsmessungen in einem Edelstahlbioreaktor mit einer Keramik-Schaumsonde. Auch Kamera- bzw. Bild-basierte Systeme werden zur Schaumerkennung eingesetzt (siehe z. B. WO 2020/198518 A1, US 11 327 064 B2, EP 3 957 712 A1), ebenso wie optische Systeme, die Lichtstreuung oder Transmission messen.

Grundsätzlich lässt sich Schaum auf chemische, mechanische und thermische Weise bekämpfen. Chemische Antischaummittel, wie etwa Silikonöle, können in unterschiedlichen Konzentrationen eingebracht werden. Die Zugabe erfolgt insbesondere im Kopfraum des Bioreaktors, von wo das Antischaummittel auf die Oberfläche des Mediums im Bioreaktor tropft. Die Zugabe ist aber auch submers möglich. Eine Vorrichtung mit einem Antischaummittelvorrat im Kopfraum des Bioreaktors, der bei Schaumkontakt Antischaummittel freigibt, ist aus der WO 2017/029259 A1 bekannt. Einrichtungen zur mechanischen Schaumzerstörung werden vor allem in Glass- oder Edelstahlbioreaktoren eingesetzt. Es gibt aber auch Lösungen für Einweg-Bioreaktoren, z. B. mittels einer Sprühdüse oder Ultraschall (siehe DE 20 2020 003 748 U1).

Der Einsatz von schaumzerstörenden oder -hemmenden Maßnahmen kann nach einem vorgegebenen zeitbasierten Profil erfolgen, das auf der Grundlage von Erfahrungswerten für den jeweiligen Prozess (subjektive optische Kontrolle und bisherige Erfahrungen bezügliche der benötigten Antischaummittelmenge) erstellt wurde. Beispielsweise kann vorgesehen sein, dass alle 60 Sekunden 1 mL Antischaummittel einer bestimmten Konzentration zugegeben wird. Antischaummaßnahmen können auch sensorbasiert erfolgen. Wenn etwa ein optisches System Schaum erkennt, wird Antischaummittel mit vorgegebener fester Pumprate in den Bioreaktor gefördert, bis das Schaumniveau unter einen kritischen Wert fällt, was z. B. mit einer Schaumsonde festgestellt werden kann. Alternativ kann Antischaummittel über ein vorgegebenes Zeitintervall in den Bioreaktor gepumpt werden, und danach wird mittels einer Kontrollschleife überprüft, ob die Zugabe ausreichend war. Wenn das Sensorsignal noch vorhanden bzw. noch zu hoch ist, wird ein weiteres Pumpintervall gestartet.

Die WO 2021/011484 A1 zeigt ein Fermenter-Steuerungssystem mit einer Vorrichtung zur Messung des Gasvolumenanteils (GVF), einem Steuergerät und einem oder mehreren Entlüftungsmechanismen. Die Steuerung ist so konfiguriert, dass sie auf der Grundlage der von der GVF-Messvorrichtung empfangenen Eingaben eine geeignete Menge an Antischaummittel bestimmt. Das Fermenter-Steuerungssystem kann ferner eine oder mehrere Hilfsmessvorrichtungen umfassen, wobei das Steuergerät dann ein Steuersignal für den Entlüftungsmechanismus auf der Grundlage der Eingaben von der GVF-Messvorrichtung und den weiteren Hilfsmessvorrichtungen erzeugt. Die Hilfsmessvorrichtungen können einen Temperatursensor, einen pH-Sensor, einen Sensor für die Mischgeschwindigkeit oder einen Durchflusssensor für die Prozess-, Eingangs- oder Rezirkulationsleitungen des Fermenters umfassen.

Aufgabe der Erfindung ist es, bei einem Bioprozess zur Kultivierung von Zellen in einem Bioreaktor eine effektivere Schaumkontrolle zu ermöglichen.

Gelöst wird diese Aufgabe durch ein Verfahren mit den Merkmalen des Anspruchs 1 und durch eine Vorrichtung mit den Merkmalen des Anspruchs 13. Vorteilhafte und zweckmäßige Ausgestaltungen des erfindungsgemäßen Verfahrens und der erfindungsgemäßen Vorrichtung sind in den zugehörigen Unteransprüchen angegeben.

Das erfindungsgemäße Verfahren dient zur Automatisierung der Schaumregelung während eines Bioprozesses zur Kultivierung von Zellen in einem Bioreaktor. Der Begriff "Zellen" umfasst hier insbesondere Säugetierzellen, Pilzzellen, Hefezellen und Bakterien. Das erfindungsgemäße Verfahren umfasst folgende Schritte: wiederholte Bestimmung von Werten einer oder mehrerer Prozessvariablen des Bioprozesses, die für die Schaumbildung im Bioreaktor relevant ist/sind; Übermitteln der bestimmten Werte als Eingangssignale an einen Controller; Erzeugen von Ausgangssignalen durch den Controller für wenigstens eine Schaumregulierungsvorrichtung in Abhängigkeit der Eingangssignale; und bedarfsgerechte Steuerung der wenigstens einen Schaumregulierungsvorrichtung mittels der vom Controller ausgegebenen Ausgangssignale.

Der Schritt "Bestimmung von Werten einer oder mehrerer Prozessvariablen des Bioprozesses, die für die Schaumbildung im Bioreaktor relevant ist" wird wiederholt durchgeführt, wobei die zeitlichen Abstände durch eine Prozesssteuerung vorgegeben sind.

Unter einer Prozessvariable ist hier ein auf den konkreten Bioprozess bezogener Prozessparameter zu verstehen, der maßgeblichen Einfluss auf die Schaumbildung hat und gemessen, berechnet oder auf sonstige Weise bestimmt wird. Eine solche Prozessvariable ist abzugrenzen von einem Signal einer dedizierten Schaumerkennungseinrichtung.

Eine "bedarfsgerechte" Steuerung der wenigstens einen Schaumregulierungsvorrichtung mittels der vom Controller ausgegebenen Ausgangssignale bedeutet z. B., dass bei chemischer Schaumbekämpfung möglichst wenig Antischaummittel eingesetzt wird, oder dass bei mechanischer Schaumzerstörung, etwa durch Rühren im Kopfraum, die entsprechende Maßnahme nicht mit höherer Intensität, als sie für einen planmäßigen weiteren Verlauf des Bioprozesses notwendig ist, durchgeführt wird.

Die Erfindung beruht auf der Erkenntnis, dass die derzeit üblichen Maßnahmen zur Schaumbekämpfung in Bioprozessen in ihrer Intensität nicht automatisch steuerbar sind, sondern - wenn überhaupt - nur manuell, was zeitaufwendig und fehlerhaft sein kann, wobei zudem keine Abstimmung auf den jeweiligen Prozess oder ein Prozessereignis vorgesehen ist. Das erfindungsgemäße Verfahren erlaubt eine feinere, genauere und damit bessere Regelung. Im Ergebnis führt dies zu signifikanten Vorteilen. Beispielsweise kann der Verbrauch von Antischaummittel reduziert werden, was deshalb von Bedeutung ist, da sich Antischaummittel negativ auf das Downstream-Processing auswirkt. Außerdem erfolgt dank der Erfindung der Einsatz von Antischaummaßnahmen gezielt und zu einem geeigneten Zeitpunkt, was die Effektivität erhöht, denn bei einem zu frühen Eingreifen wäre die Wirkung vermindert oder würde dann nachlassen. All dies ist auch vorteilhaft für die Leistungsfähigkeit bzw. Langlebigkeit der gesamten Abluftstrecke und für eine(n) bessere(n) und besser reproduzierbare(n) Gelöstsauerstoff-Verlauf/Kontrolle. Ein weiterer wesentlicher Vorteil besteht darin, dass das erfindungsgemäße Verfahren eine Schaumregelung ohne einen dedizierten Schaumsensor ermöglicht.

Gemäß einer Weiterbildung der Erfindung werden die Werte mehrerer verschiedener Prozessvariablen als mehrere Eingangssignale an den Controller übermittelt, wobei der Controller dann die Ausgangssignale in Abhängigkeit der separaten Eingangssignale zur Steuerung der wenigstens einen Schaumregulierungsvorrichtung erzeugt und ausgibt. Diese Weiterbildung ist nützlich, wenn während des Bioprozesses mehrere Prozessvariablen bestimmbar sind, die für die Schaumbildung im Bioreaktor relevant sind. Aus der Mehrzahl von Eingangssignalen können durch entsprechende Verarbeitung (Auswertungsalgorithmus) grundsätzlich genauere Rückschlüsse auf die Schaumbildung gezogen und diese Erkenntnisse in die gezielte Schaumbekämpfung einbezogen werden.

Als besonders geeignete Prozessvariablen, die für die Schaumbildung im Bioreaktor relevant sind, erweisen sich folgende: Zelldichte; Lebendzellvolumen; Lebendzelldichte; Zellwachstumsrate; Proteinkonzentration; Proteinmenge; Sauerstoffverbrauch; Wert für volumenbezogenen Stoffübergangskoeffizienten (kLa-Wert); Kohlenstoffdioxidkonzentration; Glukoseverbrauch; Nährlosungsverbrauch; Perfusionsrate, Bleedrate (Zelldichtekorrekturrate), Begasungsrate; Trübung. Dementsprechend wird bevorzugt der Wert einer oder mehrerer dieser Prozessvariablen und/oder deren zeitliche Veränderung als Input an den Controller übermittelt.

Als Controller-Input kann aber alternativ oder zusätzlich auch die Zeit ab Prozessbeginn (Batch-Zeit) oder die Zeit ab Beginn eines bestimmten Prozessschritts oder die Zeit ab einem bestimmten Prozessereignis herangezogen werden.

Einen erheblichen Einfluss auf die Schaumbildung kann auch die Verwendung eines Spargers haben. Abhängig vom Spargertyp (z. B. Mikrosparger, Makrosparger) bzw. der Lochanzahl und -größe und/oder der Begasungsrate durch den Sparger wird mehr oder weniger Schaum gebildet. Auch die Art des vom jeweiligen Spargertyp produzierten Schaums - feinporig oder grobporig, feucht oder trocken - spielt bei der bedarfsgerechten Bekämpfung eine wesentliche Rolle. Dementsprechend sieht eine bevorzugte Ausführungsform der Erfindung vor, diese Aspekte als Prozessvariable bei der Schaumregulierung zu berücksichtigen. Das bedeutet, dass der oder die aktuell im Prozess betriebenen Spargertypen und/oder dessen/deren Begasungsrate (jeweils) eine Prozessvariable im Controller ist, genauso wie z. B. die Zelldichte. So führt etwa die Verwendung eines Mikrospargers im Vergleich zu einem Makrosparger in der Regel zu mehr Schaum, was eine höhere Schaumkorrekturintensität erfordert. Im Hinblick auf einen oder mehrere "aktuell" betriebene Sparger ist zu beachten, dass ein Sparger im laufenden Prozess gewechselt werden kann, insbesondere bei den typischen Prozessen, in denen ein Kombisparger eingesetzt wird. Es kann z. B. zwischen einem Makrosparger (große Luftblasen) und einem Mikrosparger (kleine Luftblasen) o. ä. - oder einer Kombination von beiden - gewählt werden. Da der jeweils betriebene Sparger direkten Einfluss auf das Schaumverhalten im Bioreaktor hat, ist es sinnvoll, ihn mit in die Regelung einzubeziehen. Außerdem lässt sich der aktuell betriebene Sparger wieder auf gewisse Bioprozessparameter (Sauerstoffverbrauch, Zellzahl, Kultivierungsdauer etc.) zurückführen.

Die Werte der Prozessvariable(n), deren Wert(e) als Input an den Controller übermittelt werden, werden - soweit möglich - durch vorhandene Sensoren direkt gemessen, oder sie ergeben sich aus dem Ablaufprotokoll der Prozesssteuerung (z. B. verwendeter Spargertyp, Begasungsrate). Die Bestimmung des Werts bzw. der Werte für die Steuerung der Schaumregulierungsvorrichtung(en) kann aber auch indirekt erfolgen, insbesondere durch weitere Verarbeitung von Messwerten eines oder mehrerer Sensoren, etwa eines Abgassensors.

Gemäß einer besonders vorteilhaften Ausführungsform der Erfindung umfasst der Controller einen Führungsregler, dem die Werte der einen oder mehreren Prozessvariablen übermittelt werden, und wenigstens einen Folgeregler, der die wenigstens eine Schaumregulierungsvorrichtung steuert. Der wenigstens einen Schaumregulierungsvorrichtung ist ein Steuerprofil zugewiesen, das vom Ausgangssignal des Führungsreglers abhängig ist. Ein solcher Betrieb kann als "Polygonsteuerung" bezeichnet werden. Das Steuerungsprofil kann fest vorgegeben sein oder vom Nutzer voreingestellt werden.

Auf diese Weise lassen sich auch mehrere Schaumregulierungsvorrichtungen durch die Ausgangssignale des Controllers steuern, entweder gleichzeitig oder kaskadierend.

Gemäß einer Weiterbildung der Erfindung werden die Werte mehrerer verschiedener Prozessvariablen jeweils als separate Eingangssignale an mehrere Controller übermittelt. Jeder Controller erzeugt Ausgangssignale in Abhängigkeit seiner Eingangssignale zur Steuerung wenigstens einer Schaumregulierungsvorrichtung. Je nach gewählter Strategie wird ein Ausgangssignal oder werden mehrere Ausgangssignale ausgewählt, um eine oder mehrere Schaumregulierungsvorrichtungen zu steuern.

Die eine oder mehreren Schaumregulierungsvorrichtungen führen vorzugsweise wenigstens eine der folgenden Maßnahmen durch: Zugabe von Antischaummittel, bevorzugt in einstellbarer Konzentration, mittels einer durch die Ausgangssignale des Controllers gesteuerten Pumpe; mechanische Schaumzerstörung; Änderung einer Gaszufuhr durch einen oder mehrere Sparger. Hierbei ist zu beachten, dass die jeweilige Maßnahme - wie vorher erläutert - bedarfsgerecht durchgeführt wird, d. h. nicht einfach nach einer festen Vorgabe ein- und wieder ausgeschaltet wird. Im Falle der Zugabe von Antischaummittel kann dieses in Abhängigkeit von Zelldichte, Prozesszeit etc. so zugegeben werden, dass das Antischaummittel als eine Art Schutzschicht (protective layer) auf die Oberfläche des Mediums im Bioreaktor aufgebracht wird. Wenn also zu erwarten ist, dass mit steigender Zelldichte mehr Schaum entsteht, wird auf diese Weise bedarfsgerecht darauf reagiert.

Ergänzend kann vorgesehen sein, dass die Eingangssignale des Controllers zusätzlich auf Signalen von Sensoren basieren, die Schaum bzw. dessen Wachstum und/oder den Füllstand im Bioreaktor unmittelbar detektieren bzw. überwachen. Durch diese zusätzlichen Informationen lässt sich ggf. eine noch bessere Schaumregulierung erzielen.

Ebenso kann in die bedarfsgerechte Steuerung der wenigstens einen Schaumregulierungsvorrichtung ein vorher erstelltes Modell einbezogen werden. Das Modell kann dabei als Basis für einen weiteren Controller-Input oder zur Kontrolle bzw. zum Abgleich der anderen Eingangsgröße(n) herangezogen werden.

Wie bereits angedeutet, ist es besonders vorteilhaft, dass die Schaumregulierung durch die bedarfsgerechte Steuerung der wenigstens einen Schaumregulierungsvorrichtung ohne Verwendung einer dedizierten Schaumerkennungseinrichtung durchgeführt werden kann.

Die Erfindung schafft auch eine Vorrichtung zur Automatisierung der Schaumregelung während eines Bioprozesses zur Kultivierung von Zellen in einem Bioreaktor, mit einer Einrichtung zur Bestimmung von Werten einer oder mehrerer Prozessvariablen des Bioprozesses, die für die Schaumbildung im Bioreaktor relevant ist, einem Controller, an den die bestimmten Werte als Eingangssignale übermittelt werden, und einer Schaumregulierungsvorrichtung, die durch vom Controller in Abhängigkeit der Eingangssignale erzeugte Ausgangssignale gesteuert wird. Die Vorrichtung ist zur Durchführung des erfindungsgemäßen Verfahrens eingerichtet.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine schematische Skizze einer Bioreaktor-Anlage mit Sensoren und Schaumregulierungsvorrichtungen;
- Figur 2 einen grundlegenden Regelkreis einer automatisierten Schaumregelung;
- Figur 3 ein erstes Beispiel für eine Steuerprofil-Kombination;
- Figur 4 ein zweites Beispiel für eine Steuerprofil-Kombination;
- Figur 5 ein drittes Beispiel für eine Steuerprofil-Kombination; und
- Figur 6 ein viertes Beispiel für eine Steuerprofil-Kombination.

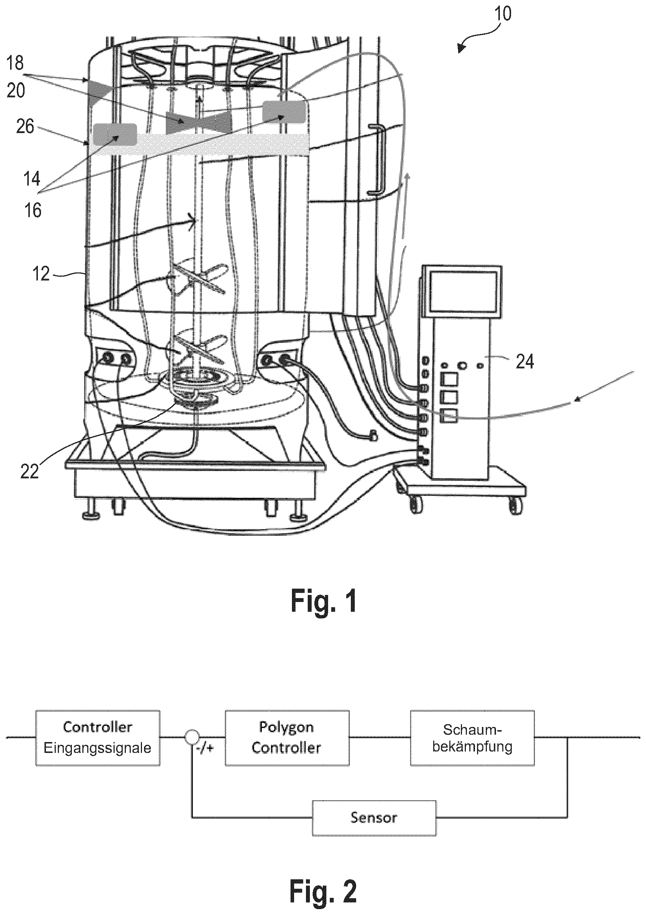

In Figur 1 ist beispielhaft eine Bioreaktor-Anlage 10 mit einem Behälter 12 gezeigt, in dem ein Medium aufgenommen ist. In dem Behälter sind unter anderem mehrere Sensoren 14, 16 und Schaumregulierungsvorrichtungen 18, 20, 22 vorgesehen.

Die Sensoren 14, 16 können jeweils zur Messung oder indirekten Bestimmung einer der folgenden Prozessvariablen dienen: Zelldichte; Lebendzellvolumen; Lebendzelldichte; Zellwachstumsrate; Proteinkonzentration; Proteinmenge; Sauerstoffverbrauch; Wert für volumenbezogenen Stoffübergangskoeffizienten (kLa-Wert); Kohlenstoffdioxidkonzentration; Glukoseverbrauch; Nährlosungsverbrauch; Perfusionsrate, Bleedrate, Begasungsrate; Trübung.

Als Schaumregulierungsvorrichtungen 18, 20, 22 sind hier eine Einrichtung zur Zugabe von Antischaummittel 18, insbesondere mithilfe einer Pumpe, und eine Einrichtung zur mechanischen Schaumzerstörung 20 vorgesehen. Außerdem werden auch die Sparger, hier ein Kombi-Sparger 22 mit zwei unterschiedlich ausgebildeten Lochkonfigurationen (Mikrosparger bzw. Makrosparger), zusätzlich als Schaumregulierungsvorrichtung genutzt.

Die Sensoren 14, 16 sowie die Schaumregulierungsvorrichtungen 18, 20 sind mit einem außerhalb des Behälters 12 angeordneten Controller 24 (Mess- und Regelungssystem) verbunden, der zudem über einen Timer zur Zeitmessung verfügt. Der Controller 24 umfasst einen oder mehrere Führungsregler, denen die Sensoren 14, 16 zugeordnet sind, sowie einen oder mehrere Folgeregler, die die Schaumregulierungsvorrichtungen 18, 20, 22 steuern. Im Controller 24 sind Steuerprofile hinterlegt, auf die später noch genauer eingegangen wird. Die Steuerprofile können fest vorgegeben sein oder vom Benutzer ausgewählt oder individuell konfiguriert werden.

Während eines laufenden Bioprozesses zur Kultivierung von Zellen bildet sich Schaum, der hier symbolisch dargestellt und mit dem Bezugszeichen 26 versehen ist. Der Schaum 26 kann verschiedene Formen und Strukturen annehmen. Der Schaum 26 kann z. B. grob- oder feinporig sein, eine(n) unterschiedliche(n) Feuchtigkeit bzw. Feuchtigkeitsgradienten haben, lokal oder flächig über dem Medium verteilt sein (von einem Impeller zentriert verteilt) etc. Diese Parameter hängen von den oben genannten Prozessvariablen ab.

Nachfolgend werden einige grundlegende Zusammenhänge zwischen einigen Prozessvariablen und deren Einfluss auf die Schaumbildung kurz erläutert, ohne Anspruch auf Vollständigkeit:
Mit viel Schaum 26 ist grundsätzlich zu rechnen, wenn
   - der Prozess bereits lange andauert;
   - die Zelldichte hoch ist (bestimmbar mit einem Zelldichtesensor bzw. Lebendzellvolumensensor);
   - viel Protein im Medium vorliegt (bestimmbar mit einem Zelldichtesensor bzw. Lebendzellvolumensensor), beispielsweise bedingt durch bestimmte Medienkomponenten, Produktbildung, "Verschmutzung" durch tote Zellen);
   - die Zellwachstumsrate hoch ist;
   - der Sauerstoffverbrauch bzw. die Kohlenstoffdioxidbildung hoch ist (deutet auf eine hohe Wachstumsrate oder hohe Zelldichte hin);
   - die pH-Wert Änderung stark ist (deutet auf starkes Wachstum oder Änderung des metabolischen Zustandes hin);
   - der Glukoseverbrauch bzw. der Mediumverbrauch hoch ist (deutet auf starkes Wachstum hin);
   - eine Induktion bzw. ein Temperaturshift vorliegt (deutet auf eine Änderung des metabolischen Zustandes der Zellen hin, z. B. wird Produktbildung molekular aktiviert; je nach Prozessart und weiteren Umständen könnte es auch sein, dass die Schaumbildung vermindert ist, da sich das Wachstum der Zellen verringert);
   - die Perfusionsrate bzw. die Bleedrate hoch ist (deutet auf eine hohe Wachstumsrate hin und hohen Mediumaustausch; der Effekt kann hier in beide Richtungen gehen, d. h. die Schaumbildung kann zunehmen oder abnehmen, je nachdem, welchen Einfluss das frische Medium hat, wobei sich die Effekte auch ausgleichen können);
   - ein Sparger mit kleinen Löchern in Betrieb ist oder die Begasungsrate hoch ist.

In Figur 2 ist ein grundlegender Regelkreis für die Schaumregelung in der in Figur 1 beispielhaft gezeigten Bioreaktoranlage 10 dargestellt. Bei der Darstellung ist zu beachten, dass alle Controller-Eingangssignale (sofern mehrere verfügbar sind) und alle Controller-Ausgangssignale, mit denen die Schaumregulierungsvorrichtungen 18, 20, 22 gesteuert werden (sofern mehrere verfügbar sind), zusammengefasst sind.

Wie bereits angedeutet, können die Schaumregulierungsvorrichtungen 18, 20, 22 eine oder mehrere der folgenden Maßnahmen zur Schaumbekämpfung ergreifen:
Es wird Antischaummittel zugegeben, bevorzugt in einstellbarer Konzentration, mittels einer durch die Ausgangssignale des Controllers 24 gesteuerten Pumpe, wobei die Pumprate (Pumpgeschwindigkeit) erhöht oder gesenkt und/oder das Zeitintervall für die Antischaummittelzugabe verlängert oder verkürzt und/oder die Konzentration des Antischaummittels erhöht oder gesenkt werden kann.

Der vorhandene Schaum 26 wird mechanisch zerstört, wobei die Intensität (Geschwindigkeit bzw. Rate) der Maßnahme erhöht oder verringert werden kann.

Die Gaszufuhr durch den/die Sparger 22 wird geändert (Auswahl des Mikrospargers oder des Makrospargers, Einstellung der Begasungsrate).

Auch ein kaskadiertes Einsetzen von mehreren Maßnahmen zur Schaumbekämpfung (nacheinander) ist grundsätzlich möglich.

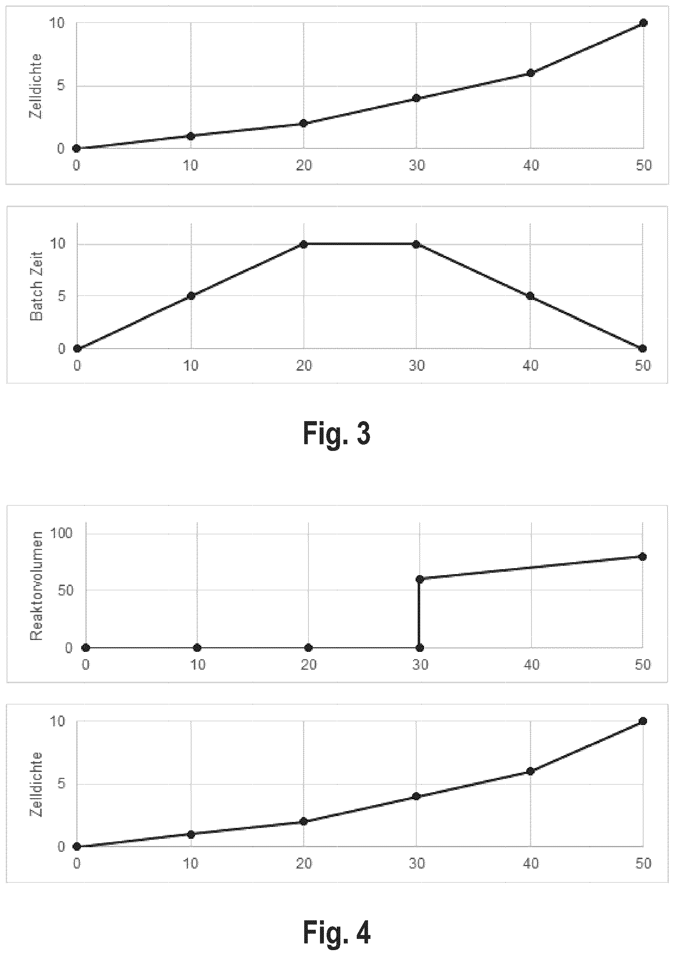

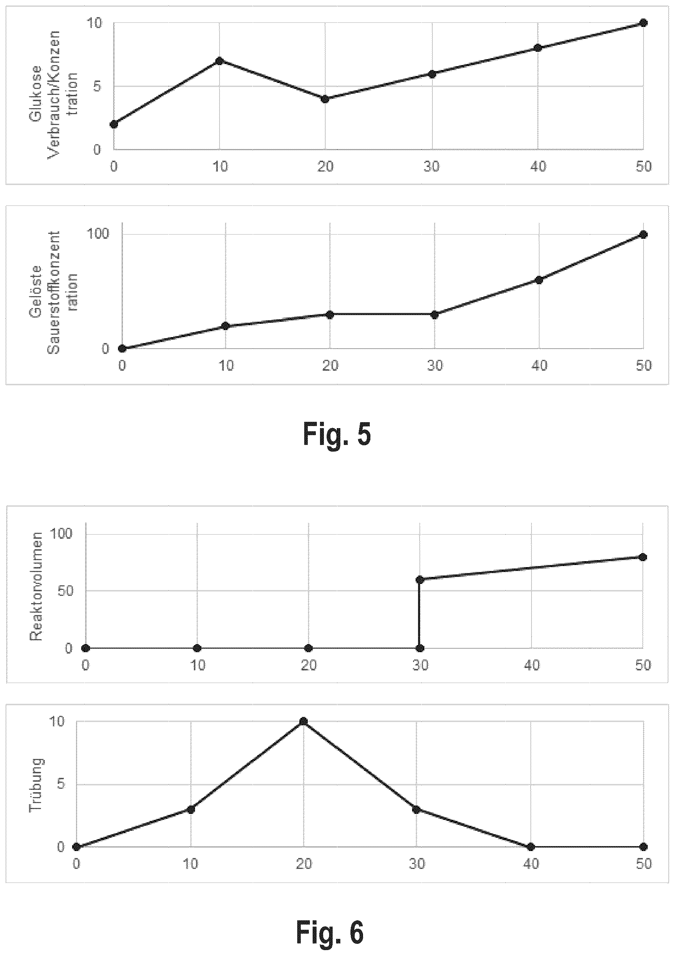

Die Figuren 3 bis 6 zeigen Beispiele für verschiedene Polygonkombinationen für die Schaumregulierung. In Figur 3 sind Zelldichte und Batch-Zeit, in Figur 4 Zelldichte und Reaktorvolumen, in Figur 5 Glukoseverbrauch/Konzentration und gelöste Gaskonzentration (z. B. Sauerstoff) und in Figur 6 Trübung und Reaktorvolumen kombiniert. Die Diagramme geben jeweils an, welches Ausgangssignal, angegeben als Prozentwert bezogen auf den Regelbereich des Führungsreglers (der interessierende Bereich liegt in den gezeigten Beispielen zwischen 0% und 50%, kann aber auch 0% bis 100% oder ein anderes Intervall sein) zur Steuerung einer oder mehrerer Schaumregulierungsvorrichtungen 18, 20, 22 ausgegeben wird. Die Schaumregelung kann vorsehen, dass einer oder mehrere Ausgangssignale zur Steuerung einer oder mehrerer Schaumregulierungsvorrichtungen 18, 20, 22 genutzt wird. Aus Anwendungssicht ist eine Schaumregelung basierend auf nur einer Prozessvariablen gegenüber einer Kombination von Prozessvariablen grundsätzlich bevorzugt, wobei das Heranziehen einer Kombination von der gewählten Strategie abhängt.

Ergänzt werden kann die Schaumregulierung durch Echtzeit-Spektroskopiemessungen und deren Auswertung mittels hinterlegter Modelle und/oder durch zusätzliche Füllstandsmessungen (z. B. mit einer ohnehin vorhandenen Kamera). Je höher der Füllstand ist, desto intensiver sollte die Schaumbekämpfung sein, wogegen bei sehr niedrigem Füllstand z. B. auf eine sofortige Antischaummittelzugabe (vorläufig noch) verzichtet werden kann. Ebenso können die Eigenschaften des Schaums 26, insbesondere Porosität (grobporig oder feinporig), Festigkeit, Feuchtigkeit, Feuchtigkeitsgradient, Ausdehnung über dem Medium, mit geeigneten Einrichtungen bestimmt und in die Schaumregulierung einbezogen werden.

### Liste der Bezugszeichen

- 10: Bioreaktor-Anlage
- 12: Behälter
- 14: erster Sensor
- 16: zweiter Sensor
- 18: erste Schaumregulierungsvorrichtung
- 20: zweite Schaumregulierungsvorrichtung
- 22: dritte Schaumregulierungsvorrichtung / Kombi-Sparger
- 24: Controller
- 26: Schaum

## Patentansprüche

1. Verfahren zur Automatisierung der Schaumregelung während eines Bioprozesses zur Kultivierung von Zellen in einem Bioreaktor, wobei das Verfahren folgende Schritte umfasst:
- wiederholte Bestimmung von Werten einer oder mehrerer Prozessvariablen des Bioprozesses, die für die Schaumbildung im Bioreaktor relevant ist/sind;
- Übermitteln der bestimmten Werte als Eingangssignale an einen Controller;
- Erzeugen von Ausgangssignalen durch den Controller für wenigstens eine Schaumregulierungsvorrichtung in Abhängigkeit der Eingangssignale;
- bedarfsgerechte Steuerung der wenigstens einen Schaumregulierungsvorrichtung mittels der vom Controller ausgegebenen Ausgangssignale.

2. Verfahren nach Anspruch 1, **dadurch gekennzeichnet, dass** die Werte mehrerer verschiedener Prozessvariablen als mehrere Eingangssignale an den Controller übermittelt werden und dass der Controller die Ausgangssignale in Abhängigkeit der separaten Eingangssignale zur Steuerung der wenigstens einen Schaumregulierungsvorrichtung erzeugt und ausgibt.

3. Verfahren nach Anspruch 1 oder 2, **dadurch gekennzeichnet, dass** die eine oder mehreren Prozessvariablen
eine oder mehrere der folgenden und/oder eine zeitliche Veränderung einer oder mehrerer der folgenden ist/sind: Zelldichte; Lebendzellvolumen; Lebendzelldichte; Zellwachstumsrate; Proteinkonzentration; Proteinmenge; Sauerstoffverbrauch; Wert für volumenbezogenen Stoffübergangskoeffizienten (kLa-Wert); Kohlenstoffdioxidkonzentration; Glukoseverbrauch; Nährlosungsverbrauch; Perfusionsrate, Bleedrate, Begasungsrate; Trübung; und/oder
die Zeit ab Prozessbeginn oder die Zeit ab Beginn eines bestimmten Prozessschritts oder die Zeit ab einem bestimmten Prozessereignis ist; und/oder
die Art eines aktuell betriebenen Spargers und/oder die über einen Sparger eingebrachte Begasungsrate ist.

4. Verfahren nach einem der vorhergehenden Ansprüche, **dadurch gekennzeichnet, dass** die Bestimmung der Werte der einen oder mehreren Prozessvariablen indirekt durch weitere Verarbeitung von Messwerten eines Sensors erfolgt, etwa eines Abgassensors.

5. Verfahren nach einem der vorhergehenden Ansprüche, **dadurch gekennzeichnet, dass** der Controller einen Führungsregler, dem die Werte der einen oder mehreren Prozessvariablen übermittelt werden, und wenigstens einen Folgeregler umfasst, der die wenigstens eine Schaumregulierungsvorrichtung steuert, wobei der Schaumregulierungsvorrichtung ein Steuerprofil zugewiesen ist, das vom Ausgangssignal des Führungsreglers abhängig ist.

6. Verfahren nach einem der vorhergehenden Ansprüche, **dadurch gekennzeichnet, dass** mehrere Schaumregulierungsvorrichtungen durch die Ausgangssignale des Controllers gesteuert werden.

7. Verfahren nach Anspruch 6, **dadurch gekennzeichnet, dass** die Ausgangssignale des Controllers verwendet werden, um kaskadierend mehrere Schaumregulierungsvorrichtungen zu steuern.

8. Verfahren nach einem der vorhergehenden Ansprüche, **dadurch gekennzeichnet, dass** die Werte mehrerer verschiedener Prozessvariablen jeweils als separate Eingangssignale an mehrere Controller übermittelt werden und dass jeder Controller Ausgangssignale in Abhängigkeit seiner Eingangssignale zur Steuerung wenigstens einer Schaumregulierungsvorrichtung erzeugt.

9. Verfahren nach einem der vorhergehenden Ansprüche, **dadurch gekennzeichnet, dass** die eine oder mehreren Schaumregulierungsvorrichtungen wenigstens eine der folgenden Maßnahmen durchführen: Zugabe von Antischaummittel, bevorzugt in einstellbarer Konzentration, mittels einer durch die Ausgangssignale des Controllers gesteuerten Pumpe; mechanische Schaumzerstörung; Änderung einer Gaszufuhr durch einen oder mehrere Sparger.

10. Verfahren nach einem der vorhergehenden Ansprüche, **dadurch gekennzeichnet, dass** die Eingangssignale des Controllers zusätzlich auf Signalen von Sensoren basieren, die Schaum bzw. dessen Wachstum und/oder den Füllstand im Bioreaktor unmittelbar detektieren bzw. überwachen.

11. Verfahren nach einem der vorhergehenden Ansprüche, **dadurch gekennzeichnet, dass** in die bedarfsgerechte Steuerung der wenigstens einen Schaumregulierungsvorrichtung ein vorher erstelltes Modell einbezogen wird.

12. Verfahren nach einem der vorhergehenden Ansprüche, **dadurch gekennzeichnet, dass** die Schaumregulierung durch die bedarfsgerechte Steuerung der wenigstens einen Schaumregulierungsvorrichtung ohne Verwendung einer dedizierten Schaumerkennungseinrichtung durchgeführt wird.

13. Vorrichtung zur Automatisierung der Schaumregelung während eines Bioprozesses zur Kultivierung von Zellen in einem Bioreaktor, mit einer Einrichtung zur Bestimmung von Werten einer oder mehrerer Prozessvariablen des Bioprozesses, die für die Schaumbildung im Bioreaktor relevant ist, einem Controller, an den die bestimmten Werte als Eingangssignale übermittelt werden, und einer Schaumregulierungsvorrichtung, die durch vom Controller in Abhängigkeit der Eingangssignale erzeugte Ausgangssignale gesteuert wird, **dadurch gekennzeichnet, dass** die Vorrichtung zur Durchführung des Verfahrens nach einem der vorhergehenden Ansprüche eingerichtet ist.
